# EUROPEAN PATENT APPLICATION

(11) **EP 4 671 257 A1**
(43) Date of publication of application: **31.12.2025**
(21) Application number: 24759690.1
(22) Date of filing: 21.02.2024
(51) Int. Cl.: C07F 9/6512, C07F 9/6561, C07H 19/10, C12N 15/113, A61K 48/00, A61K 31/713, A61P 31/12, A61P 35/00

(54) **NUCLEOTIDE ANALOG, AND PREPARATION METHOD THEREFOR AND USE THEREOF**

(30) Priority: 21.02.2023 CN 202310143263
(71) Applicant: Hitgen Inc., Chengdu, Sichuan 610200 (CN)
(72) Inventor: LI, Jin, Chengdu, Sichuan 610200 (CN); ZHANG, Shuai, Chengdu, Sichuan 610200 (CN); MA, Huiyong, Chengdu, Sichuan 610200 (CN); LIU, Guansai, Chengdu, Sichuan 610200 (CN); HU, Yongjian, Chengdu, Sichuan 610200 (CN); BO, Chongfei, Chengdu, Sichuan 610200 (CN); LUO, Huadong, Chengdu, Sichuan 610200 (CN)
(74) Representative: Vitina, Maruta
(86) International application number: PCT/CN2024/077851
(87) International publication number: WO 2024/175013

(57) **Abstract**

A lipophilic nucleotide analogue of the following formula: 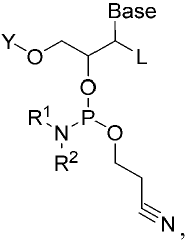 where Y is a hydroxyl protecting group, R¹ is a C₁₋₆ alkyl or a halogen-substituted C₁₋₆ alkyl, R² is a C₁₋₆ alkyl or a halogen-substituted C₁₋₆ alkyl, L is a lipophilic group; and Base is a nucleotide base. A preparation of the nucleotide analogue from 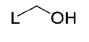 is provided. A method for enhancing the in-vivo delivery of nucleic acid drugs with the nucleotide analogue is also provided.

## Description

### TECHNICAL FIELD

This application relates to pharmaceutical technology, and more particularly to nucleotide analogues, and a preparation and application thereof.

### BACKGROUND

With the development of nucleic acid chemical synthesis technologies, nucleic acids and their modified analogs have been widely used in the fields of chemistry, biology, and medicine. Chemical modifications of nucleotides include modifications of the sugar ring, the nucleobase, or the phosphate backbone, as well as substitution of the natural nucleoside with a chemical structure having a specific function. Unnatural nucleotides include Peptide Nucleic Acids (PNA), Morpholino and Locked Nucleic Acids (LNA), Glycerol Nucleic Acids (GNA), Threose Nucleic Acids (TNA), and Unlocked Nucleic Acids (UNA).

The delivery of small interfering RNA (siRNA) to cells *in vivo* requires specific targeting and substantial protection from extracellular environments, particularly serum proteins. The siRNA therapy shows a promising potential for treating liver-related disorders. However, challenges in delivering siRNA to extrahepatic tissues limit its therapeutic application. The addition of lipophilic moieties to nucleic acid molecules is one of the earliest modification approaches to enhance cellular uptake and delivery of antisense oligonucleotides (ASOs) and siRNA to the liver and other organs. Lipophilic conjugates can improve the siRNA delivery, facilitating the uptake into alveolar and bronchiolar epithelial cells (Brown, K.M., Nair, J.K., Janas, M.M. et al. Expanding RNAi therapeutics to extrahepatic tissues with lipophilic conjugates. Nat Biotechnol (2022)). The current extrahepatic delivery strategies involve lipid nanoparticles and N-acetylgalactosamine (GaINAc) conjugates. Nevertheless, there is still a need for methods of improving the in-vivo delivery of siRNA molecules to fully enhance their therapeutic potential. In view of this, the present disclosure provides a lipophilic nucleotide analogue aimed at enhancing the in-vivo delivery efficiency of siRNA. The present disclosure also provides a method for synthesizing such nucleotide analogue with simple operation and desirable yield.

### SUMMARY

Technical solutions of the present disclosure are described as follows.

This application provides a compound of formula (I), or a stereoisomer, or a pharmaceutically acceptable salt thereof: 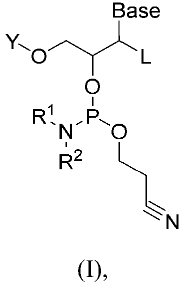
wherein Y is a hydroxyl protecting group;
R¹ is a C₁-₆ alkyl or a halogen-substituted C₁₋₆ alkyl;
R² is a C₁-₆ alkyl or a halogen-substituted C₁₋₆ alkyl;
L is a lipophilic group; and
Base is a nucleotide base.

In some embodiments, a stereoisomeric structure of the compound is represented by Formula (II): 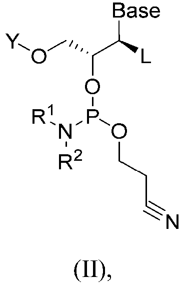
wherein Y is the hydroxyl protecting group;
R¹ is the C₁₋₆ alkyl or the halogen-substituted C₁₋₆ alkyl;
R² is the C₁₋₆ alkyl or the halogen-substituted C₁₋₆ alkyl;
L is the lipophilic group; and
the Base is the nucleotide base.

In some embodiments, L is selected from the group consisting of a cholesteryl group, a C₆-₂₀ alkyl, a C₆-₂₀ alkenyl and a C₆-₂₀ alkynyl.

In some embodiments, R¹ is isopropyl; R² is isopropyl; and L is a cholesteryl group or a C₆-₂₀ alkyl.

In some embodiments, L is selected from a C₆-₂₀ straight-chain alkyl.

In some embodiments, L is selected from the group consisting of a C₆ straight-chain alkyl, a C₇ straight-chain alkyl, a C₈ straight-chain alkyl, a C₉ straight-chain alkyl, a C₁₀ straight-chain alkyl, a C₁₁ straight-chain alkyl, a C₁₂ straight-chain alkyl, a C₁₃ straight-chain alkyl, a C₁₄ straight-chain alkyl, a C₁₅ straight-chain alkyl, a C₁₆ straight-chain alkyl, a C₁₇ straight-chain alkyl, a C₁₈ straight-chain alkyl, a C₁₉ straight-chain alkyl and a C₂₀ straight-chain alkyl.

In some embodiments, L is selected from the group consisting of

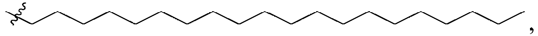

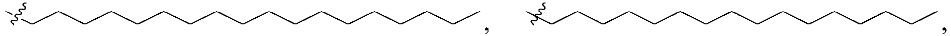

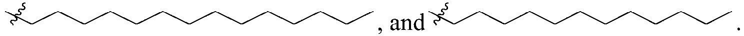

In some embodiments, the Base is selected from the group consisting of

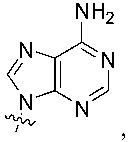

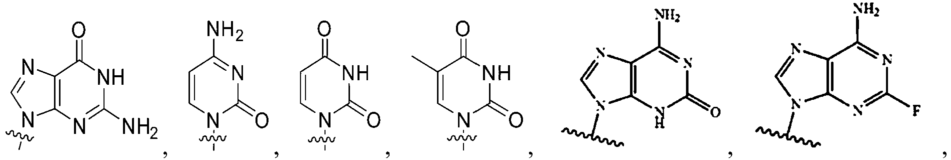

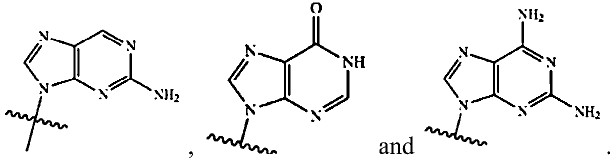

In some embodiments, Y is selected from the group consisting of 4,4'-dimethoxytrityl, 4-methoxytrityl, trityl, trimethylsilyl, triisopropylsilyl, tert-butyldimethylsilyl, triethylsilyl, phenyldimethylsilyl, benzyloxycarbonyl and 2-bromobenzyloxycarbonyl.

In some embodiments, the compound is represented by:

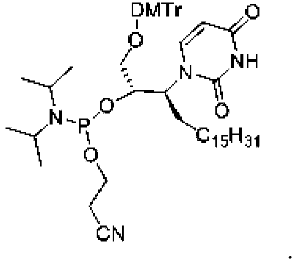

This application also provides a use of the above-described nucleotide analogues in nucleic acid delivery.

This application also provides a use of the above-described nucleotide analogues in the delivery of small interfering RNA (siRNA) and antisense oligonucleotide (ASO).

This application also provides a use of the above-described nucleotide analogues as intermediates in the preparation of nucleic acid molecules.

This application also provides a use of the above-described nucleotide analogues as intermediates in the preparation of siRNA and ASO molecule.

This application also provides a use of the above-described nucleotide analogues as intermediates in the preparation of a sense strand of siRNA.

This application also provides a use of the above-described nucleotide analogues as intermediates in the preparation of nucleotides at positions 2 to 9 from 5'-end of the sense strand of siRNA.

This application also provides a use of the above-described nucleotide analogues as intermediates in the preparation of nucleotides at position 2, 3, 4, 5, 6, 7, or 8 from the 5'-end of the sense strand of siRNA.

This application provides a method for synthesizing a nucleotide analogue, comprising:
(1) dissolving a compound A1 in dichloromethane followed by addition of Dess-Martin periodinane and NaHCO₃ and reaction at room temperature for 5-16 h to obtain a compound A2;
(2) dissolving ethyl (triphenylphosphoranylidene) acetate in dichloromethane followed by addition of a dichloromethane solution of the compound A2 under stirring and reaction at room temperature for 5-16 h to obtain a compound A3;
(3) dissolving the compound A3 in dichloromethane followed by addition of diisobutyl aluminum hydride and reaction at -10°C-5°C for 1-3 h to obtain a compound A4;
(4) dissolving tetraisopropyl titanate in dichloromethane followed by addition of diethyl D-(-)-tartrate (CAS number: 13811-71-7) under stirring at -30°C-5°C, addition of a dichloromethane solution of the compound A4 and tert-butyl hydroperoxide, and reaction for 10-24 h to obtain a compound A5;
(5) dissolving the compound A5 in pyridine followed by addition of a hydroxyl protecting group reagent and reaction at room temperature under stirring for 5-16 h to obtain a compound A6;
(6) dissolving the compound A6, a nucleotide base reagent and 1,8-diazabicyclo [5.4.0] undec-7-ene in a solvent in a microwave reaction tube followed by reaction under stirring at 90-120°C for 5-12 h to obtain a compound A7; and
(7) dissolving the compound A7 in dichloromethane followed by addition of 4,5-dicyanoimidazole and 2-cyanoethyl N,N,N',N'-tetraisopropyl-phosphordiamidite and reaction at 20-40°C for 20 min-3 h to obtain a compound A8; and

as shown in the following synthesis route: 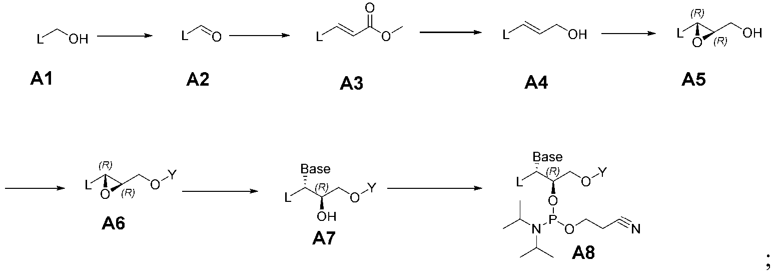
wherein Y is a hydroxyl protecting group; L is a lipophilic group; and Base is a nucleotide base.

In some embodiments, Y is 4,4'-dimethoxytrityl, L is a C₁₆ straight-chain alkyl, and the Base is uracil.

In some embodiments, in step (1), a molar equivalent ratio of the compound A1 to the Dess-Martin periodinane to NaHCO₃ is 1:1.0-1.5:2-6;
in step (2), a molar equivalent ratio of the compound A2 to ethyl (triphenylphosphoranylidene) acetate is 1:1.0-1.5;
in step (3), a molar equivalent ratio of the compound A3 to diisobutyl aluminum hydride is 1:2.0-3.0;
in step (4), a molar equivalent ratio of the compound A4 to tetraisopropyl titanate to diethyl D-(-)-tartrate is 1:1.0-1.5:1.0-1.5;
in step (5), a molar equivalent ratio of the compound A5 to the hydroxyl protecting group reagent is 1:1.0-1.5;
in step (6), a molar equivalent ratio of the compound A6 to the nucleotide base reagent to 1,8-diazabicyclo [5.4.0] undec-7-ene is 1:1.0-2.0:1.0-2.0; and
in step (7), a molar equivalent ratio of the compound A7 to 4,5-dicyanoimidazole to 2-cyanoethyl N,N,N',N'-tetraisopropyl-phosphordiamidite is 1:0.5-2.0:1.0-2.5.

In some embodiments, in step (1), a molar equivalent of the compound A1 is 1, a molar equivalent of Dess-Martin periodinane is 1.0, 1.1, 1.2, 1.3, 1.4 or 1.5, and a molar equivalent of NaHCO₃ is 2, 3, 4, 5 or 6. In some embodiments, in step (1), the molar equivalent of the Dess-Martin periodinane is 1.2, and a molar equivalent of NaHCO₃ is 5.

In some embodiments, in step (2), a molar equivalent of the compound A2 is 1, and a molar equivalent of ethyl (triphenylphosphoranylidene) acetate is 1.0, 1.1, 1.2, 1.3, 1.4 or 1.5. In some embodiments, in step (2), the molar equivalent of ethyl (triphenylphosphoranylidene) acetate is 1.3.

In some embodiments, in step (3), a molar equivalent of the compound A3 is 1, and a molar equivalent of diisobutyl aluminum hydride is 2.1, 2.2, 2.4, 2.6, 2.7, 2.8 or 3.0. In some embodiments, in step (3), the molar equivalent of diisobutyl aluminum hydride is 2.6.

In some embodiments, in step (4), a molar equivalent of the compound A4 is 1, a molar equivalent of tetraisopropyl titanate is 1.0, 1.1, 1.2, 1.3, 1.4or 1.5, and a molar equivalent of diethyl D-(-)-tartrate is 1.0, 1.1, 1.2, 1.3, 1.4 or 1.5. In some embodiments, in step (4), the molar equivalent of tetraisopropyl titanate is 1.4, and the molar equivalent of diethyl D-(-)-tartrate is 1.3.

In some embodiments, in step (5), a molar equivalent of the compound A5 is 1, and a molar equivalent of the hydroxyl protecting group reagent is 1.0, 1.1, 1.2, 1.3, 1.4 or 1.5. In some embodiments, in step (5), the molar equivalent of the hydroxyl protecting group reagent is 1.2. In some embodiments, in step (6), a molar equivalent of the compound A6 is 1, a molar equivalent of the nucleotide base reagent is 1.2, 1.3, 1.4, 1.5, 1.6, 1.7 or 1.8, and a molar equivalent of 1,8-diazabicyclo [5.4.0] undec-7-ene is 1.2, 1.3, 1.4, 1.5, 1.6, 1.7 or 1.8. In some embodiments, in step (6), the molar equivalent of the nucleotide base reagent is 1.5, and a molar equivalent of 1,8-diazabicyclo [5.4.0] undec-7-ene is 1.6.

In some embodiments, in step (7), a molar equivalent of the compound A7 is 1, a molar equivalent of 4,5-dicyanoimidazole is 0.5, 0.6, 0.7, 0.8, 0.9 or 1.0, and a molar equivalent of 2-cyanoethyl N,N,N',N'-tetraisopropyl-phosphordiamidite is 1.5, 1.6, 1.7, 1.8, 1.9, 2.0, 2.1 or 2.2. In some embodiments, in step (7), the molar equivalent of 4,5-dicyanoimidazole is 0.8, and the molar equivalent of 2-cyanoethyl N,N,N',N'-tetraisopropyl-phosphordiamidite is 1.9.

In some embodiments, in step (5), the hydroxyl protecting group reagent is selected from the group consisting of tert-butyldimethylchlorosilane, trimethylchlorosilane, tertbutyldiphenylchlorosilane, triisopropylchlorosilane, trityl chloride, 4-methoxytrityl chloride, 4,4',4'-trimethoxytrityl chloride, and 4,4'-dimethoxytrityl chloride. In some embodiments, in step (5), the hydroxyl protecting group reagent is 4,4'-dimethoxytrityl chloride.

In some embodiments, in step (6), the nucleotide base reagent is selected from the group consisting of adenine, guanine, thymine, cytosine, uracil, purine, xanthine and 2,6-diaminopurine. In some embodiments, in step (6), the nucleotide base reagent is uracil.

In some embodiments, in step (1), the reaction is performed for 5 h, 8 h, 10 h, 12 h, 14 h or 16 h. In some embodiments, in step (1), the reaction is performed for 16 h.

In some embodiments, in step (2), the reaction is performed for 5 h, 8 h, 10 h, 12 h, 14 h or 16 h. In some embodiments, in step (2), the reaction is performed for 16 h.

In some embodiments, in step (3), the reaction is performed at -10°C, -5°C, 0°C or 5°C for 1 h, 2 h or 3 h. In some embodiments, in step (3), the reaction is performed at -5°C for 1 h.

In some embodiments, in step (4), the reaction is performed at -30°C, -25°C, -10°C, 0°C or 5°C for 10 h, 12 h, 16 h, 18 h, 20 h, 22 h or 24 h. In some embodiments, in step (4), the reaction is performed at 0°C for 24 h.

In some embodiments, in step (5), the reaction is performed for 5 h, 8 h, 10 h, 12 h, 14 h or 16 h. In some embodiments, in step (5), the reaction is performed for 16 h.

In some embodiments, in step (6), the reaction is performed at 90°C, 100°C, 110°C or 120°C for 5 h, 8 h, 10 h, 12 h, 14 h or 16 h. In some embodiments, in step (6), the reaction time is performed at 110°C for 5 h.

In some embodiments, in step (7), the reaction is performed at 20°C, 25°C, 30°C or 35°C for 20 min, 0.5 h, 1 h or 2 h. In some embodiments, in step (7), the reaction is performed at 25°C for 0.5 h.

This application also provides an intermediate compound of Formula (III) for synthesizing the nucleotide analogue provided herein: 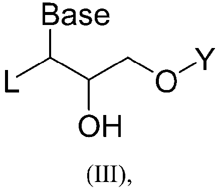 wherein Y is a hydroxyl protecting group; L is a lipophilic group; and Base is a nucleotide base.

In some embodiments, the intermediate compound is represented by Formula (IV): 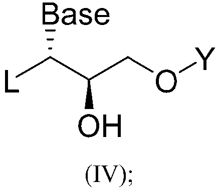
wherein Y is selected from the group consisting of 4,4'-dimethoxytrityl, 4-methoxytrityl, trityl, trimethylsilyl, triisopropylsilyl, tert-butyldimethylsilyl, triethylsilyl, phenyldimethylsilyl, benzyloxycarbonyl and 2-bromobenzyloxycarbonyl;
L is selected from the group consisting of a C₆ straight-chain alkyl, a C₇ straight-chain alkyl, a C₈ straight-chain alkyl, a C₉ straight-chain alkyl, a C₁₀ straight-chain alkyl, a C₁₁ straight-chain alkyl, a C₁₂ straight-chain alkyl, a C₁₃ straight-chain alkyl, a C₁₄ straight-chain alkyl, a C₁₅ straight-chain alkyl, a C₁₆ straight-chain alkyl, a C₁₇ straight-chain alkyl, a C₁₈ straight-chain alkyl, a C₁₉ straight-chain alkyl and a C₂₀ straight-chain alkyl; and
Base is selected from the group consisting of

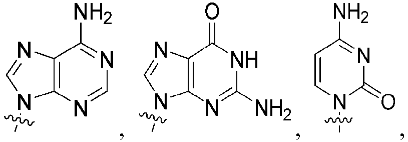

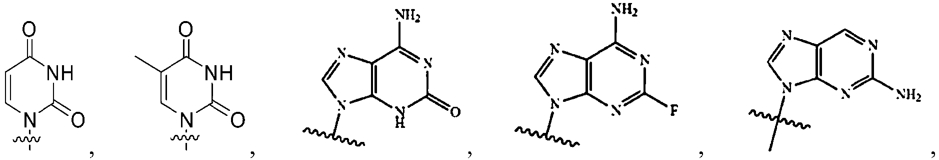

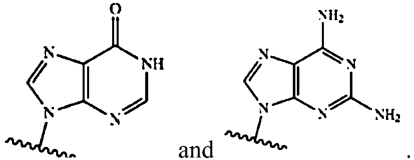

In some embodiments, Y is 4,4'-dimethoxytrityl, L is a C₁₆ straight-chain alkyl, and the Base is uracil.

In some embodiments, the intermediate compound is represented by:

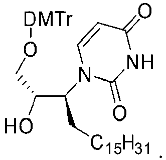

This application also provides a siRNA, comprising:
a sense strand; and
an antisense strand;
wherein the sense strand and the antisense strand each comprise 15-45 nucleotides which are each independently modified or unmodified,
the sense strand and the antisense strand are partially complementary to form a double-stranded region; and
the sense strand comprises at least one nucleotide represented by Formula (V) in the 15-45 nucleotides: 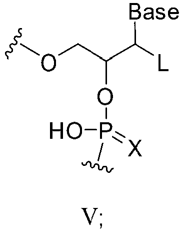
the at least one nucleotide represented by Formula (V) is covalently linked to other parts of the siRNA at 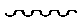 position;
wherein X is O or S;
L is a lipophilic group; and
Base is a nucleotide base.

In some embodiments, the at least one nucleotide represented by Formula (V) is represented by Formula (Va): 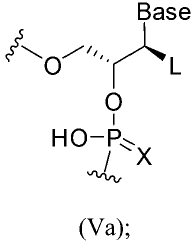
wherein X is O or S;
L is the lipophilic group; and
the Base is the nucleotide base.

In some embodiments, L is a cholesteryl group or a C₆-₂₀ alkyl.

In some embodiments, L is selected from the group consisting of

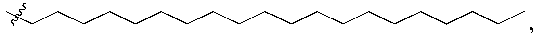

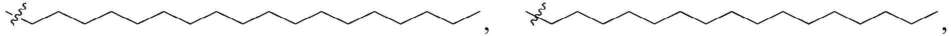

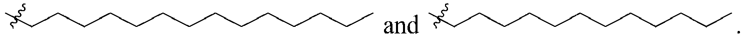

In some embodiments, the Base is selected from the group consisting of

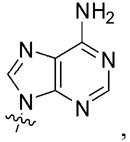

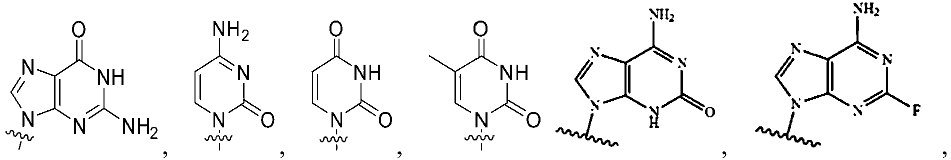

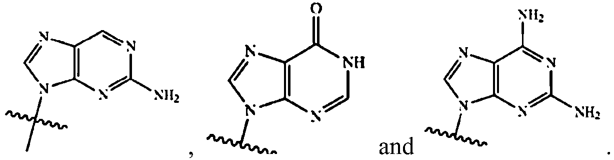

In some embodiments, the at least one nucleotide represented by Formula (V) is represented by:

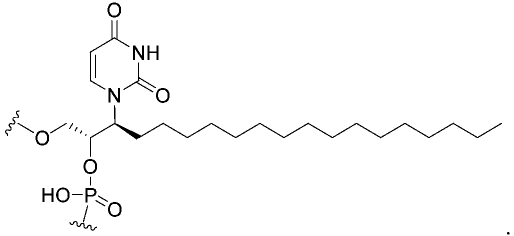

In some embodiments, the sense strand of the siRNA comprises 1, 2, 3, 4, or 5 nucleotides represented by the Formula (V). In some embodiments, the sense strand of the siRNA comprises 1 or 2 nucleotides represented by the Formula (V). In some embodiments, the sense strand of the siRNA comprises 1 nucleotide represented by the Formula (V).

In some embodiments, a nucleotide at position 2, 3, 4, 5, 6, 7, 8, 9 or 10 from 5'-end of the sense strand is represented by the Formula (V). In some embodiments, the nucleotide at position 2, 3, 4, 5, 6, 7 or 8 from the 5'-end of the sense strand is represented by the Formula (V). In some embodiments, the nucleotide at position 6 from the 5'-end of the sense strand is represented by the Formula (V).

In some embodiments, the antisense strand has a length of 19-27 nucleotides, and the sense strand has a length of 19-25 nucleotides. In some embodiments, the antisense strand has a length of 19-23 nucleotides, and the sense strand has a length of 19-21 nucleotides. In some embodiments, the antisense strand has a length of 23 nucleotides, and the sense strand has a length of 21 nucleotides.

In some embodiments, the siRNA comprises one or more single-stranded nucleotide overhangs, such as overhangs of 1, 2, 3, or 4 nucleotides. In some embodiments, the overhang(s) may be present on the sense strand, the antisense strand, or a combination thereof. In some embodiments, the overhang(s) are located at the 5'-end, the 3'-end, or both ends of the sense strand or the antisense strand of the siRNA.

In some embodiments, the 3'-end of the antisense strand of the siRNA comprises a 2-nucleotide overhang.

In some embodiments, the siRNA has blunt ends. In some embodiments, the siRNA has at least one blunt end located at the 5'- end of the antisense strand (or the 3'-end of the sense strand).

In some embodiments, the siRNA has two blunt ends.

In some embodiments, the 3'-end of the antisense strand of the siRNA comprises a 2-nucleotide overhang.

In some embodiments, the siRNA comprises at least one modified nucleotide. In some embodiments, all nucleotides in the sense strand and/or the antisense strand are modified nucleotides or nucleotide analogues.

In some embodiments, the modified nucleotides or the nucleotide analogues are independently selected from the group consisting of 2'-O-methyl nucleotide, 2'-fluoro nucleotide, 2'-deoxy nucleotide, 2',3'-seco nucleoside analogue, 2'-fluoroarabino nucleotide, 2'-O-methoxyethyl nucleotide, 2'-amino-modified nucleotide, 2'-alkyl-modified nucleotide, 3'-O-methyl nucleotide, 2'-allyl-modified nucleotide, a phosphorothioate group-containing nucleotide, a methyl phosphonate group-containing nucleotide, a 5'-phosphate group-containing nucleotide, a 5'-phosphate mimic-containing nucleotide, a diol-modified nucleotide, an abasic nucleotide, morpholino nucleotide, a locked nucleic acid, an unlocked nucleic acid and a glycerol nucleotide.

In some embodiments, the modified nucleotide of the siRNA is selected from:
2'-fluoro nucleotides at positions 7, 9, 10, and 11 from the 5'-end of the sense strand; and/or
2'-fluoro nucleotides at positions 9, 10, and 11 from the 5'-end of the sense strand.

In some embodiments, the modified nucleotide of the siRNA is selected from:
2'-fluoro nucleotides at positions 2, 14 and 16 from the 5'-end of the antisense strand; and/or
2'-fluoro nucleotides at positions 2, 6, 14 and 16 from the 5' end of the antisense strand. In some embodiments, the modified nucleotide of the siRNA is selected from:
the antisense strand comprises at least one glycerol nucleotide.

In some embodiments, the glycerol nucleotide is positioned at nucleotides 2 to 9 from the 5'-end of the antisense strand. In some embodiments, the glycerol nucleotide is positioned at nucleotides 3, 4, 5, 6, 7 or 8 from the 5'-end of the antisense strand.

In some embodiments, 5'-end and 3'-end of the sense strand each independently comprises one or two phosphorothioate groups, respectively; and/or 5'-end and 3'-end of the antisense strand each independently comprises one or two phosphorothioate groups, respectively.

In some embodiments, at least one phosphorothioate group is present between nucleotides at one or more of the following positions: between the first and second nucleotides from the 5'-end of the sense strand, between the second and third nucleotides from the 5'-end of the sense strand, between the first and second nucleotides from the 3'-end of the sense strand, between the second and third nucleotides from the 3'-end of the sense strand, between the first and second nucleotides from the 3'-end of the antisense strand, between the second and third nucleotides from the 3'-end of the antisense strand, between the first and second nucleotides from the 5'-end of the antisense strand, and between the second and third nucleotides from the 5'-end of the antisense strand. In some embodiments, at least four of the above positions comprise phosphorothioate groups. In some embodiments, at least six of the above positions comprise phosphorothioate groups. In some embodiments, eight of the above positions comprise phosphorothioate groups.

In some embodiments, phosphorothioate groups are present between the first and second nucleotides and between the second and third nucleotides from the 5'-end of the sense strand. In some embodiments, phosphorothioate groups are present between the first and second nucleotides and between the second and third nucleotides from the 5'-end of the sense strand, and between the first and second nucleotides and between the second and third nucleotides from the 3'-end of the sense strand.

In some embodiments, phosphorothioate groups are present between the first and second nucleotides and between the second and third nucleotides from the 3'-end of the antisense strand, and between the first and second nucleotides and between the second and third nucleotides from the 5'-end of the antisense strand.

In some embodiments, phosphorothioate groups are present between each of the following positions: between the first and second nucleotides and between the second and third nucleotides from the 5'-end of the sense strand; between the first and second nucleotides and between the second and third nucleotides from the 3'-end of the sense strand; between the first and second nucleotides and between the second and third nucleotides from the 3'-end of the antisense strand; and between the first and second nucleotides and between the second and third nucleotides from the 5'-end of the antisense strand.

In some embodiments, the first nucleotide at the 5'-end of the antisense strand is a nucleotide modified with an (E)-vinyl phosphate ester group.

This application also provides a pharmaceutical composition, comprising:
the siRNA described above; and a pharmaceutically acceptable carrier.

This application also provides a method for enhancing in-vivo delivery of a nucleic acid drug in a subject in need thereof, comprising:
administering the siRNA described above.

Unless otherwise specified, as used herein, the initial definition of group or term is applicable throughout the specification. And those terms that are not specifically defined herein should be construed according to the disclosure and context.

As used herein, "DMTr" refers to 4,4'-dimethoxytrityl.

As used herein, the minimum and maximum number of carbon atoms in a hydrocarbon group are indicated by prefixes. For example, the prefix "C_{a-b} alkyl" refers to any alkyl group containing from "a" to "b" carbon atoms. Thus, for instance, C₆-₂₀ alkyl refers to a straight-chain or branched alkyl group having 6-20 carbon atoms.

As used herein, the term "alkyl" refers to a straight-chain or branched hydrocarbon group derived from an alkane molecule, including but not limited to methyl (-CH₃), ethyl (-CH₂CH₃) and methylene (-CH₂-). The alkyl group may also form part of other substituents, such as C₁₋₆ alkoxy or C₁₋₆ alkylamino groups.

As used herein, the term "alkenyl" refers to a straight-chain or branched hydrocarbon group having at least two carbon atoms and at least one ethylenic unsaturated bond (>C=C<). For example, "C_{a-b} alkenyl" refers to an alkenyl group containing a to b carbon atoms, and includes, but is not limited to, vinyl, propenyl, isopropenyl and 1,3-butadienyl.

As used herein, the term "alkynyl" refers to a monovalent straight-chain or branched hydrocarbon group containing at least one carbon-carbon triple bond. The term "alkynyl" is also intended to encompass those hydrocarbon groups having a triple bond and a double bond. For example, "C₂₋₆ alkynyl" includes, but is not limited to, ethynyl and propynyl.

As used herein, the term "halogen" refers to fluorine, chlorine, bromine, or iodine.

As used herein, the term "nucleotide base" includes both naturally occurring and non-naturally occurring nucleotide bases. This should be apparent to those skilled in the art, as many nucleotide bases previously thought to be "non-naturally occurring" have since been discovered in nature. Thus, the term "nucleotide base" encompasses not only known purine and pyrimidine heterocycles, but also analogues and tautomers thereof. The nucleotide bases include, but are not limited to, adenine, guanine, thymine, cytosine, uracil, purine, xanthine, 2,6-diaminopurine, 8-oxo-N6-methyladenine, 7-deazaxanthine, 7-deazaguanine, N4,N4-ethano-bridged cytosine, N6,N6-ethano-bridged 2,6-diaminopurine, 5-methylcytosine, 5-(C₃-C₆)alkynylcytosine, 5-fluorouracil, 5-bromouracil, pseudoisocytosine, 2-hydroxy-5-methyl-4-triazolopyridine, isocytosine, isoguanine, inosine, and the "non-naturally occurring" nucleotide bases described in U.S. Patent No. 5,432,272 to Benner et al. The term "nucleotide base" includes any and all of the above examples, as well as analogues and tautomers thereof. Particularly important nucleotide bases include adenine, guanine, thymine, cytosine, and uracil, which are considered naturally occurring nucleotide bases relevant to human therapeutic and diagnostic applications. As used herein, the terms "protecting group" or "protective group" refer to labile chemical moieties known in the art to prevent undesired reactions of reactive functional groups (e.g., hydroxyl, amino, carboxyl, and thiol groups) during synthetic processes. Protecting groups are typically employed selectively and/or orthogonally to shield reactive sites while allowing reactions at other positions, and are subsequently removed to regenerate the unprotected functional group for further reactions. In some embodiments, a "substituted" group or substituent may include a protecting group.

As used herein, the term "hydroxy protecting group" includes optionally substituted trityl groups such as 4,4'-dimethoxytrityl (DMTr), 4-methoxytrityl (MMT) and trityl; optionally substituted 9-(9-phenyl)xanthenyl (pixyl); optionally substituted ethoxycarbonyloxy, p-phenylazophenoxycarbonyloxy, tetrahydropyranyl (THP), 9-fluorenylmethyloxycarbonyl (Fmoc), methoxytetrahydropyranyl (MTHP); silyloxy groups such as trimethylsilyl (TMS), triisopropylsilyl (TIPS), t-butyldimethylsilyl (TBDMS), triethylsilyl, and phenyldimethylsilyl; benzyloxycarbonyl or substituted benzyloxycarbonyl ethers such as 2-bromobenzyloxycarbonyl; tert-butyl ether; alkyl ethers such as methyl ether; acetals (including diols); acyloxy groups such as acetyl or halogen-substituted acetyl (e.g., chloroacetyl or fluoroacetyl), isobutyryl, pivaloyl, benzoyl, or substituted benzoyl; methoxymethyl (MOM); benzyl ether or substituted benzyl ethers such as 2,6-dichlorobenzyl (2,6-Cl₂Bzl).

As used herein, the term "lipophilic moiety" includes, but is not limited to, cholesterol, C₆-₂₀ alkyl groups, cholic acid, adamantaneacetic acid, 1-pyrenebutyric acid, dihydrotestosterone, 1,3-bis-O(hexadecyl)glycerol, geranyloxyhexyl, hexadecylglycerol, borneol, menthol, 1,3-propanediol, palmitic acid, myristic acid, O₃-(oleoyl) lithocholic acid, O₃-(oleoyl)cholic acid, dimethoxytrityl, or phenoxazine.

Lipophilic nucleotide analogues containing lipophilic moieties may be conjugated to siRNA through any method known in the art, including via functional groups either pre-existing in the nucleotide analogue or introduced into the siRNA, such as a hydroxyl group (e.g., -CO-CH₂-OH). Functional groups present in the nucleotide analogue or introduced into the siRNA include, but are not limited to, hydroxyl, amine, carboxyl, sulfonate, phosphate, thiol, azide group, and alkyne groups.

Conjugation of siRNA to lipophilic nucleotide analogues may occur, for example, through formation of ether, carboxyl, or carbamoyl ester groups between a hydroxyl group and an alkyl (R-), alkanoyl (RCO-), or substituted carbamoyl (RNHCO-) moiety. The alkyl group R may be cyclic (e.g., cyclohexyl) or non-cyclic (e.g., straight-chain or branched; saturated or unsaturated). Representative alkyl groups (R-) include but are not limited to butyl, pentyl, hexyl, heptyl, octyl, nonyl, decyl, undecyl, dodecyl, tridecyl, tetradecyl, pentadecyl, hexadecyl, heptadecyl, or octadecyl.

As used herein, the terms "nucleic acid" and "nucleic acid molecule" refer to polymers of linked nucleosides, each of nucleosides may be independently modified or unmodified, including oligonucleotide sequences of about 10-50 single-stranded nucleotides or double-stranded nucleotide base pairs. In some embodiments, the oligonucleotide has a base sequence at least partially complementary to a core sequence of a target gene expressed in a cell. In some embodiments, the oligonucleotide is capable of regulating the expression of the corresponding target gene after being delivered into a cell expressing the gene. The expression of the target gene may be regulated *in vitro* or *in vivo.* The terms "nucleic acid" and "nucleic acid molecule" include, but are not limited to, single-stranded antisense oligonucleotides, small interfering RNA (siRNA), double-stranded RNA (dsRNA), microRNA (miRNA), short hairpin RNA (shRNA), ribozymes, interfering RNA molecules, and dicer-substrate RNAs.

As used herein, the term "siRNA" refers to RNA or RNA-like (e.g., chemically modified RNA) oligonucleotide molecules capable of sequence-specifically reducing or inhibiting the translation of messenger RNA (mRNA).

siRNA can exert its effects through the RNA interference mechanism (e.g., by inducing mRNA degradation via interaction with the RNA interference pathway machinery in mammalian cells, such as the RNA-induced silencing complex (RISC)), or through any other mechanisms or pathways. Although the term "siRNA drug" used herein is primarily understood to act through the RNA interference mechanism, the siRNA drug is not limited or restricted to any specific pathway or mechanism. The siRNA drugs include but are not limited to: single-stranded antisense oligonucleotides, siRNA, dsRNA, miRNA, shRNA and dicer-substrate RNA. As used herein, the siRNA drugs include oligonucleotide strands having at least partial complementarity to a target mRNA. In some embodiments, the siRNA drugs are double-stranded, consisting of an antisense strand and a sense strand at least partially complementary to the antisense strand. As used herein, the terms "silencing", "reducing", "inhibiting", "downregulating" or "knocking down" refer to a decrease or reduction in the level of expression of a gene when a cell, tissue, organ, or animal is administered with an siRNA drug molecule as described herein, as compared to the expression level of the same gene in a cell, tissue, organ, or animal that has not been treated in this manner.

As used herein, the term "sequence" or "nucleotide sequence" refers to the order or arrangement of nucleobases or nucleotides, represented in alphabetical form according to standard nucleotide nomenclature and typically expressed in the 5' to 3' direction.

As used herein, the term "complementary" when used to describe the relationship between a first nucleotide sequence (e.g., the sense strand of an siRNA agent or a target mRNA) and a second nucleotide sequence (e.g., a single-stranded antisense oligonucleotide or the antisense strand of a double-stranded siRNA agent), refers to the ability of an oligonucleotide or polyoligonucleotides including the first sequence to hybridize under certain conditions (e.g., under physiological conditions in a mammalian cell or comparable in vitro conditions) with an oligonucleotide or polyoligonucleotides including the second sequence via base pairing to form a double-stranded or double-helical structure. Complementary sequences include Watson-Crick base pairing or non-Watson-Crick base pairing, and may include natural or modified nucleotides or nucleotide analogues, to the extent that such pairing supports the required hybridization. For purposes of determining identity or complementarity, the monomers *a* and *Af* are considered complementary to *U* (or *T*), and equivalent to *A.*

As used herein, the term "sense strand" refers to a strand of an RNA molecule that carries the nucleotide sequence encoding the amino acid sequence of a protein. It is also referred to as the coding strand, the positive-sense strand, or the plus strand. The nucleotide sequence that is complementary to the sense strand is referred to as the "antisense strand".

As used herein, the term "antisense strand" refers to a nucleotide sequence that is substantially or essentially reverse complementary to a segment of the mRNA sequence of a target gene having the same length as the antisense strand.

The compounds and compositions described herein may include atoms (e.g., N, O, or S) that are in a protonated or deprotonated state depending on the environment of the compound or composition. As used herein, the disclosed structures are intended to encompass compounds and compositions in which certain functional groups, such as OH, SH, or NH groups, may be either protonated or deprotonated. The present disclosure is intended to encompass such compounds and compositions regardless of their protonation or deprotonation state under a given environmental pH, as would be readily understood by those skilled in the art.

As used herein, the term "stereoisomer" includes an enantiomer, a diastereomer or a combination thereof.

As used herein, the term "pharmaceutically acceptable" refers to a carrier, delivery agent, diluent, excipient, and/or resulting salt that is generally chemically or physically compatible with other components of a given pharmaceutical formulation and are physiologically compatible with the receptor.

As used herein, the terms "salt" and "pharmaceutically acceptable salt" refer to acid-addition or base-addition salts formed between the above-described compound or its stereoisomers and inorganic and/or organic acids or bases. These terms also include zwitterionic (inner) salts and quaternary ammonium salts, such as alkylammonium salts. Such salts may be obtained directly during the final separation and purification of the compound, or may be formed by mixing the compound or its stereoisomer with an appropriate (e.g., equimolar) amount of acid or base. These salts may be precipitated and collected by filtration, recovered after solvent evaporation, or prepared by reaction in an aqueous medium and lyophilization.

The present disclosure will be further illustrated with reference to embodiment. It is obvious that described herein are merely some embodiments of the present disclosure, rather than all embodiments of the present disclosure. It should be understood that those changes and modifications made without departing from the scope of the present disclosure shall fall within the scope of the present disclosure defined by the appended claims.

### BRIEF DESCRIPTION OF THE DRAWINGS

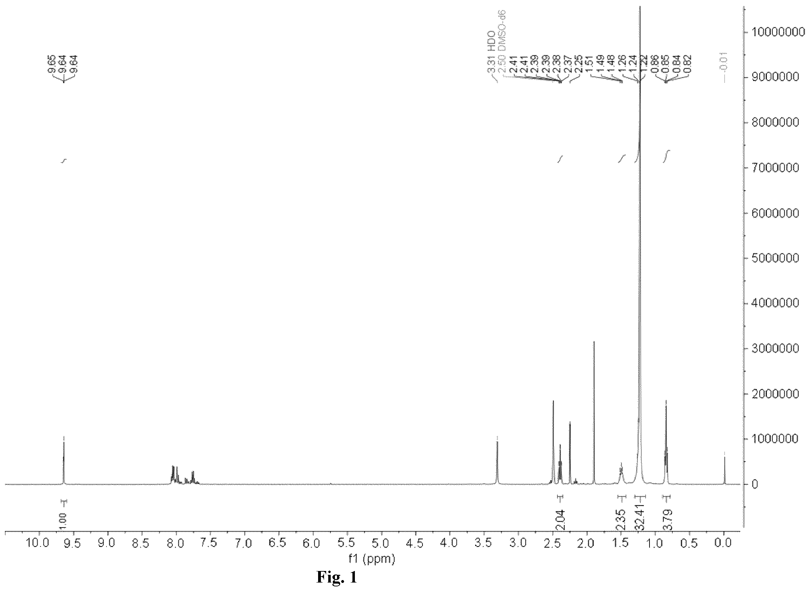

Fig. 1 is a H-Nuclear Magnetic Resonance (H-NMR) spectrum of compound a2;

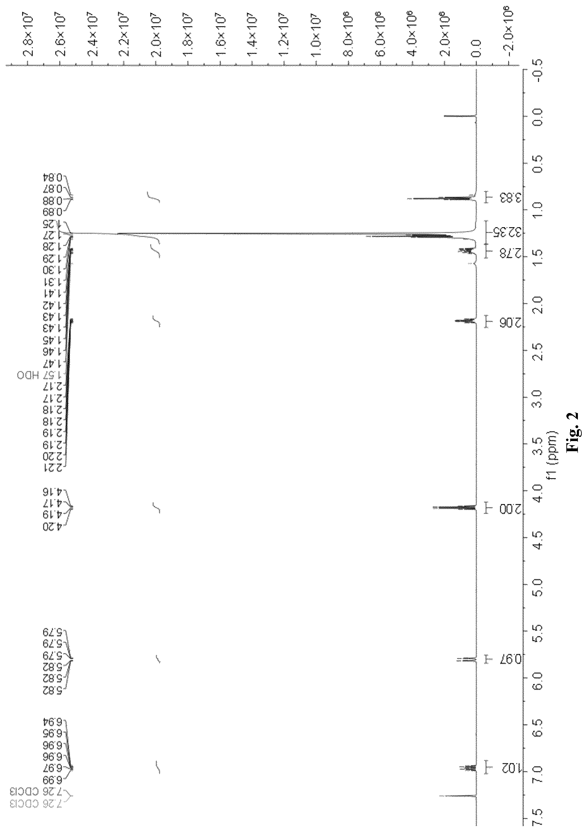

Fig. 2 is a H-NMR spectrum of compound a3;

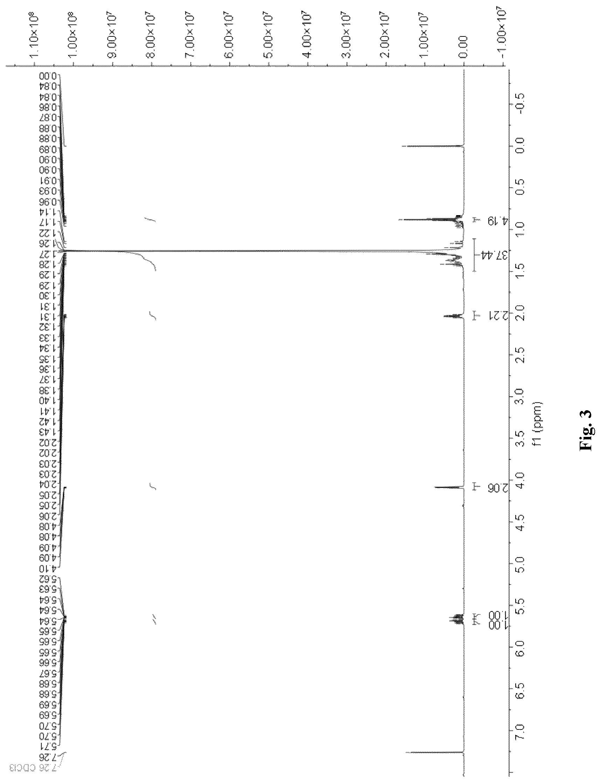

Fig. 3 is a H-NMR spectrum of compound a4;

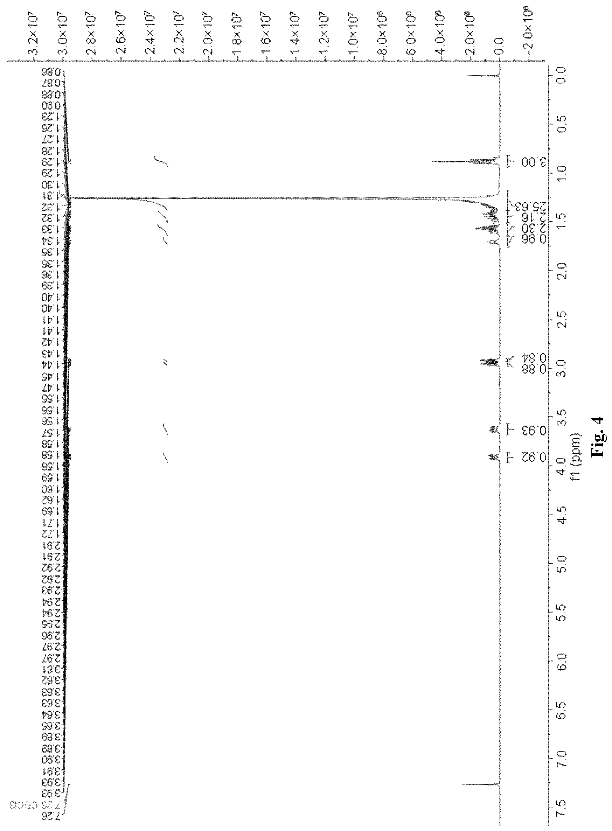

Fig. 4 is a H-NMR spectrum of compound a5;

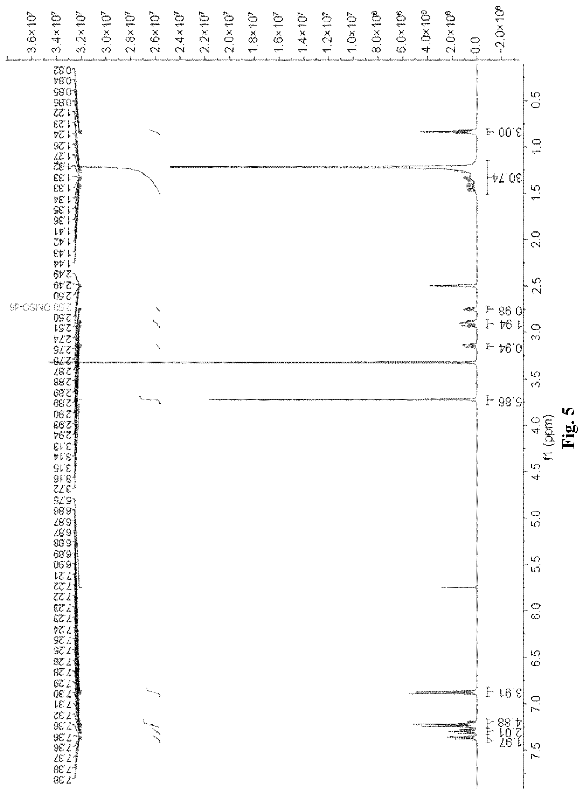

Fig. 5 is a H-NMR spectrum of compound a6;

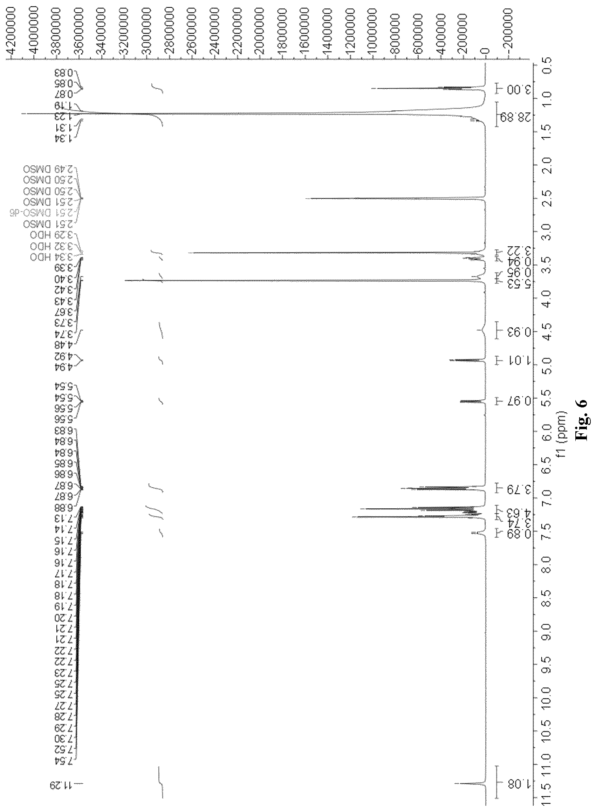

Fig. 6 is a H-NMR spectrum of compound a7;

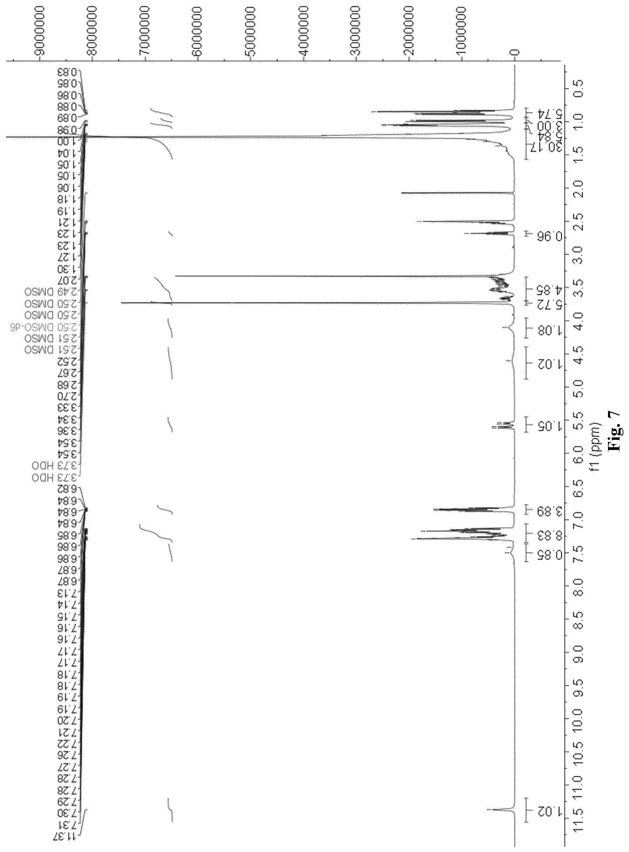

Fig. 7 is a H-NMR spectrum of compound a8;

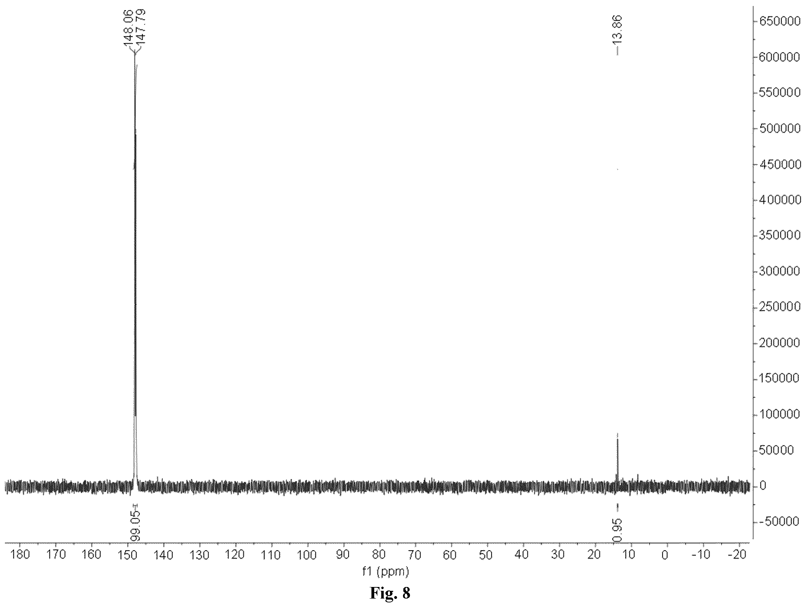

Fig. 8 is a P-Nuclear Magnetic Resonance (P-NMR) spectrum of the compound a8;
Fig. 9 shows *in vitro* activity analysis results of small interfering RNA (siRNA) synthesized in Example 2 of the present disclosure;
Fig. 10 shows the mRNA expression levels of superoxide dismutase 1 (SOD1) in various rat brain tissues on day 7 following administration of the siRNA synthesized in Example 2 of the present disclosure; and
Fig. 11 shows the SOD1 mRNA expression levels in various rat brain tissues on day 14 following administration of the siRNA synthesized in Example 2 of the present disclosure.

### DETAILED DESCRIPTION OF EMBODIMENTS

The embodiments of the present disclosure will be described in detail below with reference to the accompanying drawings. It is obvious that described herein are merely some embodiments of the present disclosure, rather than all embodiments of the present disclosure. It should be understood that various modifications, changes and replacements made by those skilled in the art without departing from the spirit of the disclosure shall fall within the scope of the present disclosure defined by the appended claims.

All raw materials, reagents and devices used in the present disclosure are commercially available. DMP: Dess-Martin periodinane; DIBAL-H: Diisobutyl aluminum hydride; DMTrCl: 4,4'-dimethoxytrityl chloride; DBU: 1,8-Diazabicyclo[5.4.0]undec-7-ene; DCI: 4,5-Dicyanoimidazole and CEP: 2-cyanoethyl N,N,N',N'-tetraisopropyl-phosphordiamidite. Unless otherwise specified, room temperature refers to the optimal reaction temperature, i.e., 20 °C-30 °C. "M" refers to mol/L.

### EXAMPLE 1 Synthesis of compound a8

### (1) Synthesis of compound a2

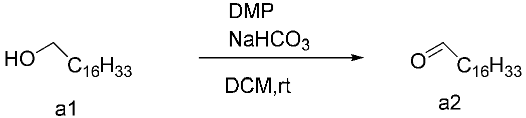

To a 2,000 mL round-bottom flask filled with nitrogen were added DMP (174.50 g, 411.55 mmol) and NaHCO₃ (144.00 g, 1714.29 mmol), followed by addition of dichloromethane (400 mL). The resulting mixture was stirred, added dropwise with a dichloromethane solution (500 mL) of 1-heptadecanol (compound **a1**) (88.00 g, 343.08 mmol), and reacted under stirring at room temperature for 16 h.

After 16 h, The thin layer chromatography (TLC) analysis showed that there was no starting material **a1** left in the reaction mixture. The reaction mixture was cooled in an ice-salt bath under stirring for 10 min, and then filtered through a diatomaceous earth bed into a clean 2,000 mL round-bottom flask. The filtration was repeated 3-4 times. The resultant filtrates were combined and concentrated to afford a crude white powder as compound **a2** (110.00 g, 79.0% yield).

### (2) Synthesis of compound a3

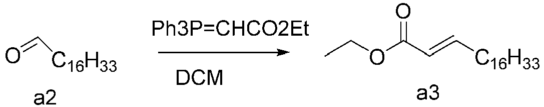

To a 2,000 mL round-bottom flask were sequentially added ethyl (triphenylphosphoranylidene) acetate (155.7 g, 446.90 mmol) and dichloromethane (500 mL). The resulting mixture was stirred, added dropwise with a dichloromethane solution (500 mL) of the compound **a2** (110 g, 79% yield, 343.70 mmol), and reacted under stirring at room temperature for 16 h.

After 16 h, the TLC analysis confirmed the complete consumption of the compound **a2.** The reaction mixture was concentrated, and purified by column chromatography using an eluent system (petroleum ether (PE): dichloromethane (DCM)=8:1). Fractions containing the desired product were collected, combined and concentrated to afford a white powder as compound **a3** (64 g, 80.0% yield).

### (3) Synthesis of compound a4

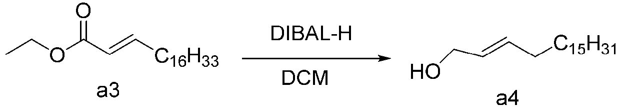

To a 2,000 mL three-neck round-bottom flask equipped with a thermometer and filled with nitrogen were sequentially added the compound **a3** (64 g, 80% yield, 158.02 mmol) and dichloromethane (700 mL). The resulting mixture was cooled to -5°C in an ice-salt bath, added dropwise with diisobutyl aluminum hydride (414.00 mL, 1 M in hexanes) (the temperature was kept below 0 °C during the whole dropwise addition process), and reacted under stirring at - 5 °C for 1 h.

After 1 h, the TLC analysis showed complete consumption of the compound **a3.** At -5 °C, the nitrogen gas was removed and replaced with a vent to a waste gas outlet. A saturated solution of potassium sodium tartrate (50 mL) was slowly added dropwise to the reaction mixture, and reacted under stirring at -5 °C for 1 h. The resulting mixture was filtered through a diatomaceous earth bed, washed with purified water and saturated brine. The organic layer was dried over anhydrous sodium sulfate and concentrated. The resulting white power was purified by column chromatography using an eluent system (PE: DCM= 2:1). Fractions containing the desired product were collected, combined and concentrated to yield a white power as compound **a4** (42 g, 85% yield).

### (4) Synthesis of compound a5

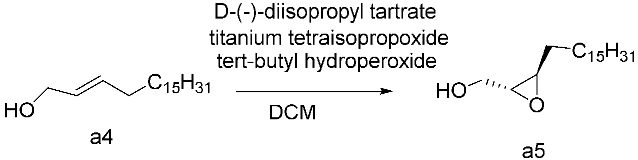

To a 250 mL three-neck round-bottom flask equipped with a thermometer and filled with nitrogen was added tetraisopropyl titanate (4.374 g, 15.40 mmol), followed by addition of dichloromethane (30 mL). The resulting mixture was cooled to -25 °C using a low-temperature circulating reactor, added with diethyl D-(-)-tartrate (4.23 g, 20.53 mmol), and reacted under stirring for 20 min. The resulting mixture was dropwise added with a dichloromethane solution (30 mL) of the compound a4 (3.62 g, 85% yield, 10.9 mmol), reacted under stirring for 20 min, and dropwise added with tert-butyl hydroperoxide (6.87 mL, 5.6 M in decane). The resulting mixture was stored in a refrigerator for 24 h.

After 24 h, the TLC analysis confirmed complete consumption of the compound **a4.** Dimethyl sulfide was added dropwise to the resulting mixture, and reacted under stirring at -9 °C for 30 min. The reaction mixture was filtered through a diatomaceous earth bed, and added with saturated sodium sulfate solution. The organic phase was washed three times with diethyl ether, then with saturated brine, dried over anhydrous sodium sulfate, and concentrated. The resulting crude product was purified by column chromatography using an eluent system (PE: DCM= 1:4). Fractions containing the desired **a5** were collected, combined and concentrated to afford a colorless oil as compound **a5** (1.42 g, 98% yield).

### (5) Synthesis of compound a6

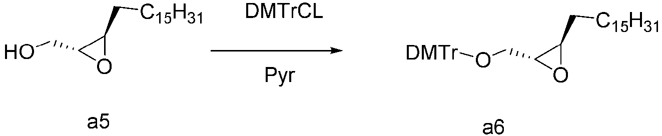

To a 100 mL round-bottom flask filled with nitrogen was added the compound **a5** (1.64 g, 5.50 mmol), followed by addition of pyridine (30 mL). The resulting mixture was stirred, slowly added with 4,4'-dimethoxytrityl chloride (2.23 g, 6.59 mmol), and reacted under stirring at room temperature for 16 h.

After 16 h, the TLC analysis confirmed that compound **a5** was completely consumed. The reaction mixture was concentrated, and purified by medium-pressure preparative chromatography using a C18 column to afford a pale yellow oily as compound **a6** (2.31 g, 98.0% yield).

### (6) Synthesis of compound a7

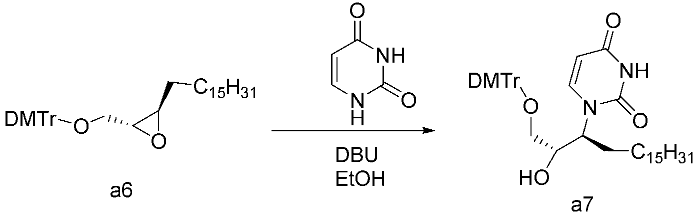

To a 50 mL microwave reaction tube were sequentially added the compound a6 (1.5 g, 2.49 mmol), uracil (0.42 g, 3.75 mmol) and 1,8-diazabicyclo[5.4.0]undec-7-ene (0.6 g, 3.94 mmol), followed by addition of anhydrous ethanol (24 mL). The reaction mixture was placed in a microwave reactor and reacted under stirring at 110 °C for 8 h.

After 8 h, the LC-MS analysis showed that 65% of the starting material had been converted to the desired product. The reaction mixture was diluted with dichloromethane, washed with purified water and saturated brine, and dried over anhydrous sodium sulfate. The organic phase was concentrated, and purified by column chromatography using an eluent system (DCM: ethyl acetate (EA=1:1, and 1% triethylamine (TEA)). Fractions containing the desired product were collected, combined and concentrated to afford a white foamy solid (1.27 g). The product was further purified by chiral separation using supercritical fluid chromatography (SFC) to afford a white foamy solid as compound **a7** (480 mg, 97% yield).

### (7) Synthesis of compound a8

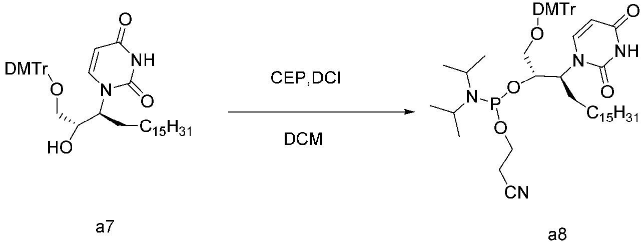

To a 25 mL round-bottom flask filled with nitrogen were added the compound **a7** (0.37 g, 0.52 mmol), followed by addition of dichloromethane (6 mL). The resulting solution was stirred, sequentially added with 4,5-dicyanoimidazole (0.05 g, 0.42 mmol) and 2-cyanoethyl N,N,N',N'-tetraisopropyl-phosphordiamidite (0.3 g, 0.99 mmol), and reacted under stirring at room temperature for 30 min.

After 30 min, the Liquid Chromatography-Mass Spectrometry (LC-MS) analysis indicated that 90% of the starting material **a7** had been converted to the desired product. The reaction mixture was concentrated under reduced pressure, and purified by medium-pressure preparative chromatography using a C18 column to afford a white foamy solid as compound **a8** (320 mg, 98% yield).

### EXAMPLE 2

The deoxynucleoside controlled pore glass (CPG) was used as the solid-phase support for the sense and antisense strands of siRNA sequences of the present disclosure. The sense strand was synthesized on a solid-phase support, while the antisense strand was synthesized using universal CPG.

A 48-channel synthesizer was employed to synthesize the sequences on a 0.2 µmol scale. The phosphoramidite monomers and the compound a8 were used at a concentration of 0.05 M, and an activator 5-Benzylthio-1H-tetrazole (BTT) was used at a concentration of 0.3 M.

The cleavage and deprotection of the synthesized sequences were performed in a 1.5 mL tube. In a first step, AMA (a mixture of ammonium hydroxide and methylamine) was used. For sequences that were fully modified at the 2'-position, ammonolysis with aqueous ammonia was performed. After cleavage and deprotection, the sequences were precipitated using a mixture of acetone and ethanol (acetone: ethanol=80:20), and then dissolved in RNase-free water. Each sequence was analyzed by LC-MS to verify sequence integrity, quantified by ultraviolet (UV) spectrophotometry, and assessed for purity using High-Performance Liquid Chromatography (HPLC).

After purification by HPLC, lyophilization, and quality inspection, sodium acetate-ethanol precipitation was used for salt exchange, followed by desalting with a 3 kDa ultrafiltration tube. The sense and antisense strands were quantified by UV spectrophotometry, mixed at a 1:1 molar ratio, and annealed to form the following double-stranded siRNAs.

| Number | Type | Sequence (5'→3') | SEQ ID NO |
|---|---|---|---|
| Hitgen-C16 | Sense strand | mC*mA*mUmUmUUghfAmAfUfCfCmUmC mAmCmUmCmUmA*mA*mA | 1 |
| | Antisense strand | VPmU*fU*mUmAmGmAgnGmUmGmAmG mGmAfUmUfAmAmAmAmUmG*mA*mG | 2 |
| Negative | Sense strand | AAGACGACUCGAAAUCCACAU | 3 |
| control | Antisense strand | GUGGAUUUCGAGUCGUCUUAA | 4 |
| C, G, U, and A denote bases of nucleotide, which may be either modified or unmodified; "m" indicates that the nucleotide immediately following the "m" is a 2'-O-methyl nucleotide; "f" indicates that the nucleotide immediately following the "f' is a 2'-fluoro nucleotide; "gn" indicates that the nucleotide immediately following the "gn" is a glycerol nucleotide (GNA); an asterisk (*) indicates that there was a phosphorothioate group between two adjacent nucleotides on two sides of the asterisk; "VP" indicates that the nucleotide immediately following the "VP" is a nucleotide modified with an (E)-vinyl phosphate ester group; and "Ugh" represents a nucleotide derived from compound a8 of the present disclosure. | | | |

To demonstrate the beneficial effects of the present disclosure, the following experimental examples were provided.

### Experimental example 1 In vitro activity analysis

### 1. Cell culture

(1) After receiving primary mouse hepatocytes (PMH), cell viability and count were assessed by staining with 0.04% trypan blue.
(2) Based on the density of viable cells and the number of wells needed, the required volumes of cell suspension and culture medium were calculated and thoroughly mixed to prepare the final cell suspension. Cells (1 × 10⁵) were seeded into 24-well plates coated with rat tail collagen, each well containing 500 µL of seeding medium (dulbecco's modified eagle medium (DMEM) supplemented with 5% fetal bovine serum, 1% penicillin-streptomycin solution, 5 µg/mL dexamethasone and 0.1% insulin-transferrin-selenium-sodium pyruvate (ITSA)). After incubation at 37°C for 3 h, cell attachment was observed under a microscope. If cells had not yet adhered, the incubation time was extended accordingly.
(3) Once cells adhered, the medium was replaced with 450 µL of complete medium (HepatoZYME-SFM supplemented with 5 µg/mL dexamethasone and 0.1% ITSA).

### 2. Cell free uptake

(1) Double-stranded siRNA was diluted to the desired concentrations using the complete cell culture medium.
(2) The prepared siRNA-containing medium was added to the 24-well plates containing cultured primary hepatocytes (50 µL per well, resulting in final siRNA concentrations of 10 nM, 100 nM, and 500 nM in the medium). The cells were then returned to the CO₂ incubator for further incubation for the designated time.

### 3. RNA extraction

RNA was extracted from the cultured cells using the Trizol method. Each sample was finally dissolved in 20 µL of RNase-free water.

### 4. Real-time quantitative PCR (qPCR)

Quantitative PCR (qPCR) was performed using PowerUp^{™} SYBR^{™} Green Master Mix Kit (Cat: A25742, Applied Biosystems Inc.) following the manufacturer's instructions. Real-time fluorescence PCR was carried out on an ABI QuantStudio^{™} 6 Real-Time PCR System using the ΔΔCt method. Each double-stranded siRNA was tested in three independent transfection experiments, with each experiment conducted in triplicate.

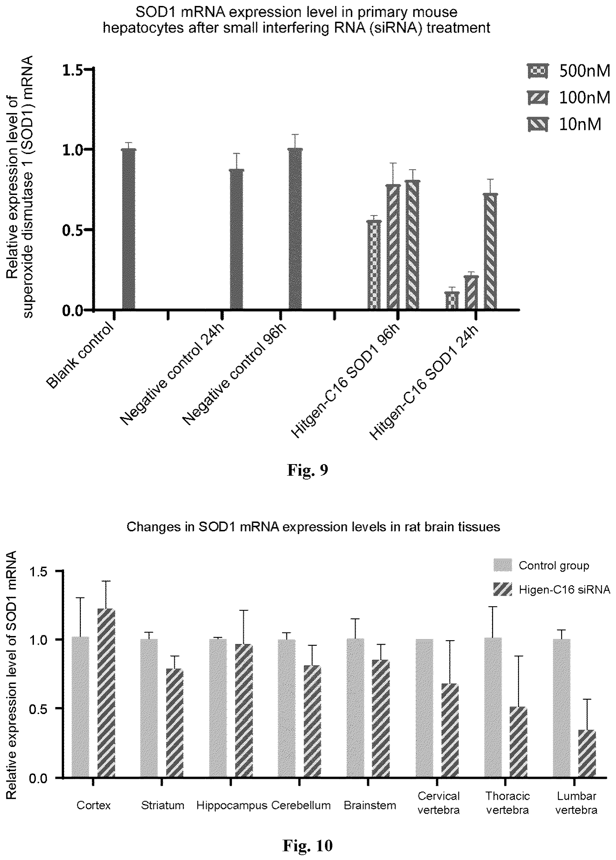

The test results for the double-stranded siRNA Hitgen-C16 described herein were shown in Fig. 9. The experimental data demonstrated that the siRNA synthesized using the nucleotide analogues of the present disclosure exhibited excellent efficacy in reducing SOD1 mRNA expression.

### Experimental example 2 small interfering RNA (siRNA) stability test

### 1. Brain homogenate stability assay

(1) 190 µL of brain homogenate was mixed with 10 µL of 20 µM double-stranded siRNA and incubated at 37 °C for 24 h. This step was performed in triplicate and served as a 24-h time point sample.
(2) 190 µL of brain homogenate was taken in triplicate and incubated simultaneously at 37 °C under identical conditions for 24 h, followed by addition of 10 µL of 20 µM double-stranded siRNA. These samples served as 0-h time point controls.
(3) 150 µL of samples from steps (1) and (2) were taken, diluted with dilution buffer and internal standard, vortexed and allowed to stand for 30 min.
(4) A solid-phase extraction (SPE) cartridge was activated, equilibrated, loaded with the sample, eluted, and the eluate was collected and evaporated under a nitrogen stream.
(5) The samples were analyzed using an ion-pairing system consisting of hexafluoroisopropanol (HFIP), N,N-diisopropylethylamine (DIEA), and ethylenediaminetetraacetic acid (EDTA).
(6) The remaining amount of siRNA after 24 h of incubation was calculated based on the analytical results.

### 2. Cerebrospinal fluid stability assay

(1) 38 µL of cerebrospinal fluid was mixed with 2 µL of 20 µM double-stranded siRNA and incubated at 37°C for 24 h. This was repeated in triplicate and designated as a 24-hour time point sample.
(2) 38 µL of cerebrospinal fluid was taken, repeated in triplicate, and incubated under identical conditions simultaneously for 24 h, followed by addition of 2 µL of 20 µM double-stranded siRNA, designated as a 0-hour time point sample.
(3) 30 µL of samples from steps (1) and (2) were taken, diluted with dilution buffer and internal standard, vortexed, then phenol-chloroform was added and vortexed again.
(4) The samples from step (3) were centrifuged, and the supernatant was collected for analysis.
(5) Sample analysis was conducted using an ion-pairing system consisting of HFIP, DIEA, and EDTA.
(6) The remaining amount of siRNA after 24 h of incubation was calculated based on the analysis results.

The siRNA test data of the present disclosure were as follows.

**Table 1. Stability test data in cerebrospinal fluid**

| Strand Name | Remaining amount at 24 h (%) | | |
|---|---|---|---|
| Hitgen-C16 sense strand | 89.20 | 98.41 | 98.82 |
| Hitgen-C16 antisense strand | 85.90 | 99.81 | 98.27 |

**Table 2. Stability Test data in brain homogenate**

| Strand Name | Remaining amount at 24 h (%) | | |
|---|---|---|---|
| Hitgen-C16 sense strand | 81.04 | 77.57 | 82.63 |
| Hitgen-C16 antisense strand | 79.64 | 72.75 | 79.82 |

The experimental data demonstrated that both the sense and antisense strands of the siRNA synthesized using the nucleotide analogues of the present disclosure exhibit good stability.

### Experimental example 3 In vivo activity assay of siRNA

Pharmacodynamic evaluation in brains of SD rats
(1) Model: Male Sprague-Dawley (SD) rats (D000017), 6 weeks old, approximately 250 g, were provided by GemPharmatech Co., Ltd.
(2) Grouping: After 7 days of acclimatization, the rats were randomly assigned into groups based on body weight, with the grouping day was designated as D0.
(3) Administration: Drug administration was initiated on D1. The dosing volume was 30 µL per rat. The route of administration was intrathecal injection via the lumbar spine, and the dosing frequency was once. (Administration was carried out in a biosafety cabinet. Prior to administration, the relevant equipment was sprayed and wiped with 75% ethanol, followed by UV irradiation for 30 min. Afterward, RNase AWAY^{™} decontamination reagent was sprayed and wiped on all relevant equipment. Surgical instruments were soaked in 75% ethanol for 30 min, then soaked and wiped with RNase AWAY^{™}. An RNase-free environment was maintained during the administration process. Before and after each administration, RNase AWAY^{™} was appropriately sprayed and wiped on the rats, equipment, instruments, and the parts of personnel entering the biosafety cabinet. The rats were weighed before administration, and their status was observed before and after administration.)
(4) Study Endpoint
   (a) On Day 7 post-administration, half of the rats in each group were euthanized. The following tissues were collected: cortex, cerebellum, brainstem, hippocampus, striatum and spinal cord (divided into cervical vertebrae, thoracic vertebrae, and lumbar vertebrae). All tissues were divided into two portions, rapidly frozen in liquid nitrogen, and transferred to -80 °C for storage.
   (b) On Day 14 post-administration, the remaining rats in each group were euthanized. The following tissues were collected: cortex, cerebellum, brainstem, hippocampus, striatum and spinal cord (divided into cervical vertebrae, thoracic vertebrae, and lumbar vertebrae). All tissues were divided into two portions, snap-frozen in liquid nitrogen and stored at -80 °C.
(5) RNA extraction: Total RNA was extracted from the collected tissues using the Trizol method. Each RNA sample was finally dissolved in 200 µL of RNase-free water.
(6) Real-Time Quantitative PCR was performed using the PowerUp^{™} SYBR^{™} Green Master Mix kit (Cat: A25742, Applied Biosystems Inc.) following the manufacturer's instructions. Real-time fluorescence PCR was carried out on the ABI QuantStudio^{™} 6 Real-Time PCR System using the ΔΔCt method.

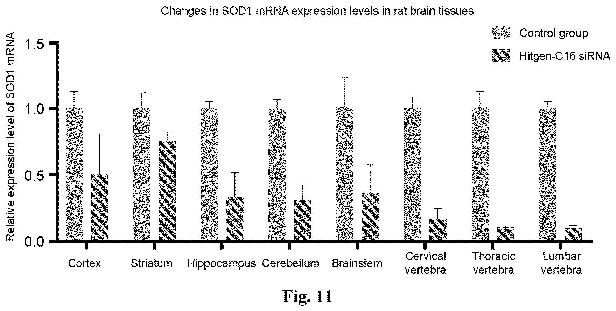

The double-stranded siRNA Hitgen-C16 developed in the present disclosure was tested, and the results were shown in Figs 10-11. The experimental data demonstrated that the siRNA synthesized using the nucleotide analogues provided herein successfully entered brain tissues and showed excellent efficacy in reducing SOD1 mRNA expression.

In summary, the nucleotide analogues provided herein can be used to enhance the *in vivo* delivery of nucleic acid therapeutics, particularly siRNA, and are especially effective in facilitating delivery to brain tissues, thereby exerting corresponding biological functions.

## Claims

1. A compound of formula (I), or a stereoisomer, or a pharmaceutically acceptable salt thereof: 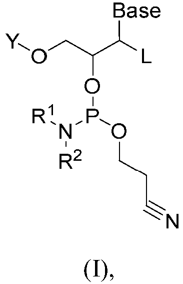
**characterized in that** Y is a hydroxyl protecting group;
R¹ is a C₁₋₆ alkyl or a halogen-substituted C₁₋₆ alkyl;
R² is a C₁₋₆ alkyl or a halogen-substituted C₁₋₆ alkyl;
L is a lipophilic group; and
Base is a nucleotide base.

2. The compound according to claim 1, **characterized in that** R¹ is isopropyl;
R² is isopropyl; and
L is a cholesteryl group or a C₆-₂₀ alkyl.

3. The compound according to claim 1, **characterized in that** L is selected from the group consisting of

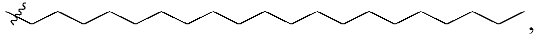

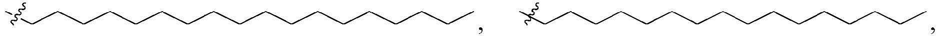

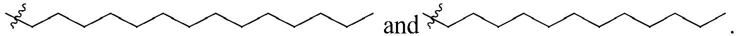

4. The compound according to claim 1, **characterized in that** the Base is selected from the group consisting of 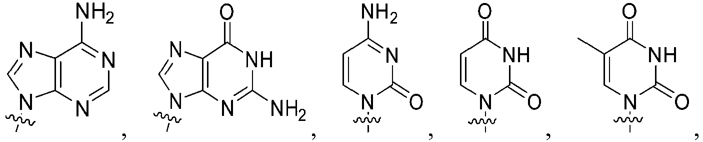 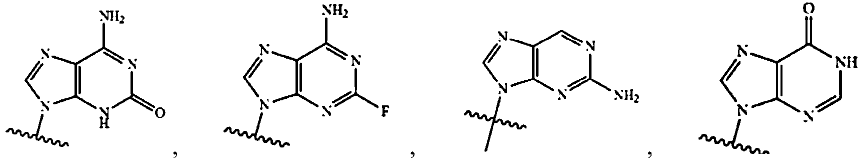 and

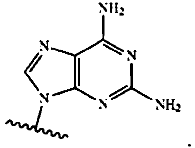

5. The compound according to claim 1, **characterized in that** Y is selected from the group consisting of 4,4'-dimethoxytrityl, 4-methoxytrityl, trityl, trimethylsilyl, triisopropylsilyl, tert-butyldimethylsilyl, triethylsilyl, phenyldimethylsilyl, benzyloxycarbonyl and 2-bromobenzyloxycarbonyl.

6. The compound according to any one of claims 1-5, **characterized in that** the compound is represented by:

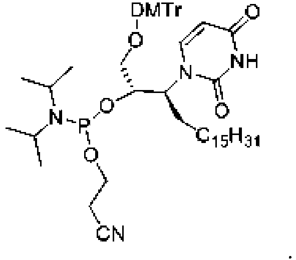

7. A method for synthesizing a nucleotide analogue, comprising:
(1) dissolving a compound A1 in dichloromethane followed by addition of Dess-Martin periodinane and NaHCO₃ and reaction at room temperature for 5-16 h to obtain a compound A2;
(2) dissolving ethyl (triphenylphosphoranylidene) acetate in dichloromethane followed by addition of a dichloromethane solution of the compound A2 under stirring and reaction at room temperature for 5-16 h to obtain a compound A3;
(3) dissolving the compound A3 in dichloromethane followed by addition of diisobutyl aluminum hydride and reaction at -10°C-5°C for 1-3 h to obtain a compound A4;
(4) dissolving tetraisopropyl titanate in dichloromethane followed by addition of diethyl D-(-)-tartrate under stirring at -30°C-5°C, addition of a dichloromethane solution of the compound A4 and tert-butyl hydroperoxide, and reaction for 10-24 h to obtain a compound A5;
(5) dissolving the compound A5 in pyridine followed by addition of a hydroxyl protecting group reagent and reaction at room temperature under stirring for 5-16 h to obtain a compound A6;
(6) dissolving the compound A6, a nucleotide base reagent and 1,8-diazabicyclo [5.4.0] undec-7-ene in a solvent in a microwave reaction tube followed by reaction under stirring at 90-120°C for 5-12 h to obtain a compound A7; and
(7) dissolving the compound A7 in dichloromethane followed by addition of 4,5-dicyanoimidazole and 2-cyanoethyl N,N,N',N'-tetraisopropyl-phosphordiamidite and reaction at 20-40°C for 20 min-3 h to obtain a compound A8; as shown in the following synthesis route:

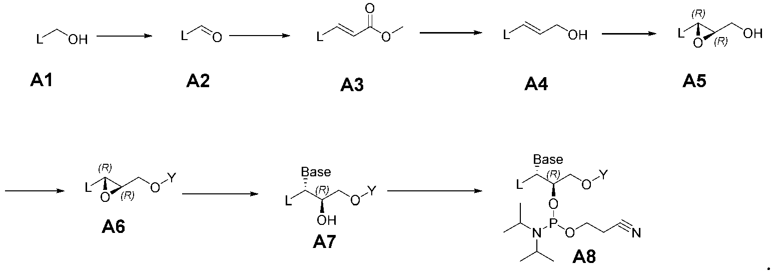

8. The method according to claim 7, **characterized in that** in step (1), a molar equivalent ratio of the compound A1 to the Dess-Martin periodinane to NaHCO₃ is 1:1.0-1.5:2-6;
in step (2), a molar equivalent ratio of the compound A2 to ethyl (triphenylphosphoranylidene) acetate is 1:1.0-1.5;
in step (3), a molar equivalent ratio of the compound A3 to diisobutyl aluminum hydride is 1:2.0-3.0;
in step (4), a molar equivalent ratio of the compound A4 to tetraisopropyl titanate to diethyl D-(-)-tartrate is 1:1.0-1.5:1.0-1.5;
in step (5), a molar equivalent ratio of the compound A5 to the hydroxyl protecting group reagent is 1:1.0-1.5;
in step (6), a molar equivalent ratio of the compound A6 to the nucleotide base reagent to 1,8-diazabicyclo [5.4.0] undec-7-ene is 1:1.0-2.0:1.0-2.0; and
in step (7), a molar equivalent ratio of the compound A7 to 4,5-dicyanoimidazole to 2-cyanoethyl N,N,N',N'-tetraisopropyl-phosphordiamidite is 1:0.5-2.0:1.0-2.5.

9. The method according to claim 7, **characterized in that** in step (1), a molar equivalent of the compound A1 is 1, a molar equivalent of the Dess-Martin periodinane is 1.0, 1.1, 1.2, 1.3, 1.4 or 1.5, and a molar equivalent of NaHCO₃ is 2, 3, 4, 5 or 6;
in step (2), a molar equivalent of the compound A2 is 1, and a molar equivalent of ethyl (triphenylphosphoranylidene) acetate is 1.0, 1.1, 1.2, 1.3, 1.4 or 1.5;
in step (3), a molar equivalent of the compound A3 is 1, and a molar equivalent of diisobutyl aluminum hydride is 2.1, 2.2, 2.4, 2.6, 2.7, 2.8 or 3.0;
in step (4), a molar equivalent of the compound A4 is 1, a molar equivalent of tetraisopropyl titanate is 1.0, 1.1, 1.2, 1.3, 1.4 or 1.5, and a molar equivalent of diethyl D-(-)-tartrate is 1.0, 1.1, 1.2, 1.3, 1.4 or 1.5;
in step (5), a molar equivalent of the compound A5 is 1, and a molar equivalent of the hydroxyl protecting group reagent is 1.0, 1.1, 1.2, 1.3, 1.4 or 1.5;
in step (6), a molar equivalent of the compound A6 is 1, a molar equivalent of the nucleotide base reagent is 1.2, 1.3, 1.4, 1.5, 1.6, 1.7 or 1.8, and a molar equivalent of 1,8-diazabicyclo [5.4.0] undec-7-ene is 1.2, 1.3, 1.4, 1.5, 1.6, 1.7 or 1.8; and
in step (7), a molar equivalent of the compound A7 is 1, a molar equivalent of 4,5-dicyanoimidazole is 0.5, 0.6, 0.7, 0.8, 0.9 or 1.0, and a molar equivalent of 2-cyanoethyl N,N,N',N'-tetraisopropyl-phosphordiamidite is 1.5, 1.6, 1.7, 1.8, 1.9, 2.0, 2.1 or 2.2.

10. The method according to claim 7, **characterized in that** in step (5), the hydroxyl protecting group reagent is 4,4'-dimethoxytrityl chloride; and
in step (6), the nucleotide base reagent is uracil.

11. The method according to claim 7, **characterized in that** in step (1), the reaction is performed for 5 h, 8 h, 10 h, 12 h, 14 h or 16 h;
in step (2), the reaction is performed for 5 h, 8 h, 10 h, 12 h, 14 h or 16 h;
in step (3), the reaction is performed at -10°C, -5°C, 0°C or 5°C for 1 h, 2 h or 3 h;
in step (4), the reaction is performed at -30°C, -25°C, -10°C, 0°C or 5°C for 10 h, 12 h, 16 h, 18 h, 20 h, 22 h or 24 h;
in step (5), the reaction is performed for 5 h, 8 h, 10 h, 12 h, 14 h or 16 h;
in step (6), the reaction is performed at 90°C, 100°C, 110°C or 120°C for 5 h, 8 h, 10 h, 12 h, 14 h or 16 h; and
in step (7), the reaction is performed at 20°C, 25°C, 30°C or 35°C for 20 min, 0.5 h, 1 h or 2 h.

12. A small interfering RNA (siRNA), comprising:
a sense strand; and
an antisense strand;
**characterized in that** the sense strand and the antisense strand each comprise 15-45 nucleotides which are each independently modified or unmodified;
the sense strand and the antisense strand are partially complementary to form a double-stranded region; and
the sense strand comprises at least one nucleotide represented by Formula (V) in the 15-45 nucleotides: 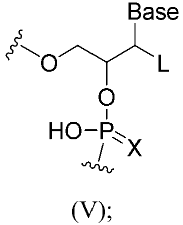
the at least one nucleotide represented by Formula (V) is covalently linked to other parts of the siRNA at 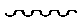 position;
wherein X is O or S;
L is a lipophilic group; and
Base is a nucleotide base.

13. The siRNA according to claim 12, **characterized in that** the at least one nucleotide represented by Formula (V) is represented by Formula (Va): 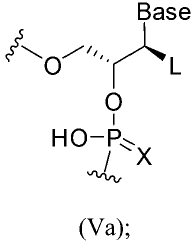 wherein X, L and the Base are defined as in claim 12.

14. The siRNA according to any one of claims 12-13, **characterized in that** L is a cholesteryl group or a C₆-₂₀ alkyl.

15. The siRNA according to claim 14, **characterized in that** L is selected from the group consisting of

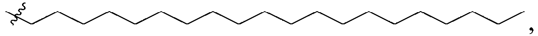

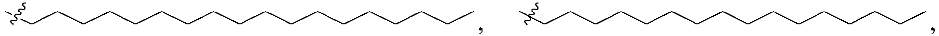

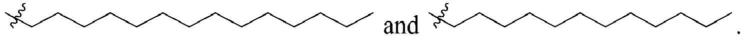

16. The siRNA according to any one of claims 12-13, **characterized in that** the Base is selected from the group consisting of 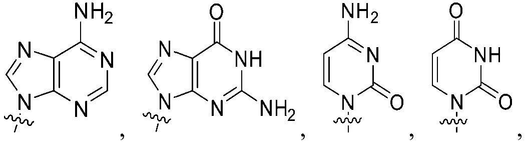 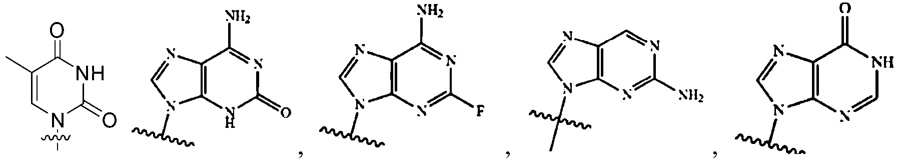 and

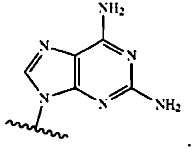

17. The siRNA according to any one of claims 12-16, **characterized in that** the at least one nucleotide represented by Formula (V) is represented by:

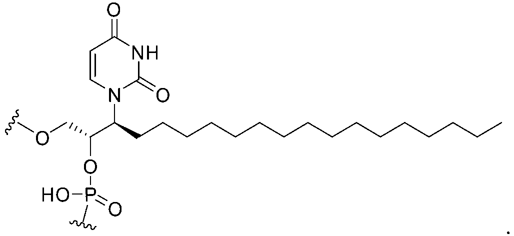

18. The siRNA according to any one of claims 12-17, **characterized in that** a nucleotide at position 2, 3, 4, 5, 6, 7, 8, 9 or 10 from 5'-end of the sense strand is represented by the Formula (V); preferably, the nucleotide at position 2, 3, 4, 5, 6, 7 or 8 from the 5'-end of the sense strand is represented by the Formula (V); and more preferably, the nucleotide at position 6 from the 5'-end of the sense strand is represented by the Formula (V).

19. The siRNA according to any one of claims 12-18, **characterized in that** the antisense strand has a length of 19-27 nucleotides, and the sense strand has a length of 19-25 nucleotides; preferably, the antisense strand has a length of 19-23 nucleotides, and the sense strand has a length of 19-21 nucleotides; and more preferably, the antisense strand has a length of 23 nucleotides, and the sense strand has a length of 21 nucleotides.

20. The siRNA according to any one of claims 12-18, **characterized in that** the siRNA comprises at least one modified nucleotide; preferably, all nucleotides in the sense strand and/or the antisense strand are modified nucleotides or nucleotide analogues.

21. The siRNA according to claim 20, **characterized in that** the modified nucleotides or the nucleotide analogues are independently selected from the group consisting of 2'-O-methyl nucleotide, 2'-fluoro nucleotide, 2'-deoxy nucleotide, 2',3'-seco nucleoside analogue, 2'-fluoroarabino nucleotide, 2'-O-methoxyethyl nucleotide, 2'-amino-modified nucleotide, 2'-alkyl-modified nucleotide, 3'-O-methyl nucleotide, 2'-allyl-modified nucleotide, a phosphorothioate group-containing nucleotide, a methyl phosphonate group-containing nucleotide, a 5'-phosphate group-containing nucleotide, a 5'-phosphate mimic-containing nucleotide, a diol-modified nucleotide, an abasic nucleotide, a morpholino nucleotide, a locked nucleic acid, an unlocked nucleic acid and a glycerol nucleotide.

22. The siRNA according to claim 20, **characterized in that** 5'-end and 3'-end of the sense strand each independently comprises one or two phosphorothioate groups; and/or
5'-end and 3'-end of the antisense strand each independently comprises one or two phosphorothioate groups.

23. A pharmaceutical composition, comprising:
the siRNA according to any one of claims 12-22; and
a pharmaceutically acceptable carrier.

24. The siRNA according to any one of claims 12-22 or the pharmaceutical composition according to claim 23 for use in the preparation of a drug.
